(19) Europäisches Patentamt
European Patent Office
Office européen des brevets

(11) Publication number : **0 361 831 B1**

(12) **EUROPEAN PATENT SPECIFICATION**

(45) Date of publication of patent specification : **22.01.92 Bulletin 92/04**

(51) Int. Cl.$^5$ : **A61K 31/70**

(21) Application number : **89309728.7**

(22) Date of filing : **25.09.89**

(54) **Antiviral nucleoside combination.**

(30) Priority : **26.09.88 GB 8822546**

(43) Date of publication of application : **04.04.90 Bulletin 90/14**

(45) Publication of the grant of the patent : **22.01.92 Bulletin 92/04**

(84) Designated Contracting States : **AT BE CH DE FR GB IT LI LU NL SE**

(56) References cited :
**EP-A- 0 206 497**
**EP-A- 0 236 935**
**CHEMICAL ABSTRACTS, vol. 110, no. 7, 13th February 1989, pages 15-16, abstract no. 50717y, Columbus, Ohio, US; R. VINCE et al.: "Potent and selective activity of a new carbocyclic nucleoside analog (carbovir: NSC 614846) against human immunodeficiency virus in vitro" & BIOCHEM. BIOPHYS. RES. COMMUN. 1988, 156(2), 1046-53**

(73) Proprietor : **THE WELLCOME FOUNDATION LIMITED**
**Unicorn House 160 Euston Road**
**London NW1 2BP (GB)**

(72) Inventor : **Barry, David Walter**
**1810 South Lakeshore**
**Drive, Chapel Hill North Carolina 27514 (US)**
Inventor : **St. Clair, Martha Heider**
**Route 1 Box 172-F**
**Rougemont North Carolina 27572 (US)**

(74) Representative : **Garrett, Michael et al**
**The Wellcome Research Laboratories Group**
**Patents and Agreements Langley Court**
**Beckenham Kent BR3 3BS (GB)**

**EP 0 361 831 B1**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to synergistic antiviral combinations of nucleoside derivatives, pharmaceutical formulations containing said combinations and their use in medical therapy, particularly in the treatment or prophylaxis of virus infections, especially retrovirus infections.

Acquired immunodeficiency syndrome, (AIDS) is an immunosuppressive or immunodestructive disease that predisposes subjects to fatal opportunistic infections. Characteristically, AIDS is associated with a progressive depletion of T-cells, especially the helper-inducer subset bearing the $OKT^4$ surface marker.

Human immunodeficiency virus (HIV) has been reproducibly isolated from patients with AIDS or with the symptoms that frequently precede AIDS. HIV is cytopathic and appears to preferentially infect and destroy T-cells bearing the OKT4 marker. It is now generally recognized that HIV is the etiological agent of AIDS.

Since the discovery that HIV is the etiological agent of AIDS, numerous proposals have been made for anti-HIV chemotherapeutic agents that may be effective in treating AIDS sufferers. Thus, for example, European Patent Application 206497 describes 2′,3′-dideoxycytidine (DDC), as being useful for the treatment of human retroviral infections including AIDS. European Patent Application 236935 describes certain carbocyclic purine nucleosides for use against virus infections including HIV infections. Certain nucleoside analogs and their use against HIV were described by Vince et al., Antiviral Research, 9 (1.2), 120 (1988). The carbocyclic purine nucleoside, (+ –)-9-(cis-4-(hydroxymethyl)-2- cyclopentenyl)guanine (NSC-614846), also known as carbovir, was disclosed by Vince at the Second International Conference on Antiviral Research, Williamsburg, VA, 10-14 April 1988."

Another group of viral pathogens of major consequence worldwide are the hepatitis viruses, in particular hepatitis B virus (HBV).

Clinical effects of infection with HBV range from headache, fever, malaise, nausea, vomiting, anorexia and abdominal pains. Replication of the virus is usually controlled by the immune response, with a course of recovery lasting weeks or months in humans, but infection may be more severe leading to persistent chronic liver disease. In "Viral Infections of Humans" (second edition, Ed., Evans, A.S. (1982) Plenum Publishing Corporation, New York), Chapter 12 described in detail the aetiology of viral hepatitis infections.

We have now discovered that the carbocyclic purine nucleoside carbovir, in combination with certain dideoxynucleosides results in a surprisingly large potentiation of the anti-HIV activity of the compounds. The use of carbovir in conjunction with the dideoxynucleosides produces a synergistic increase in anti-HIV activity in comparison with the anti-HIV activities of the individual compounds.

According to a first feature of the present invention there is provided a combination of (a) (+ –)-9-(cis-4-(hydroxymethyl)-2-cyclopentenyl)guanine (carbovir) of formula I :

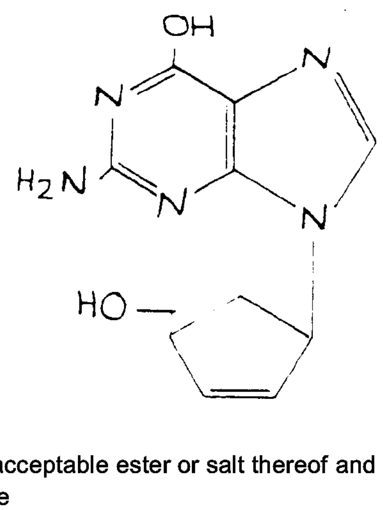

(I)

or a pharmaceutically acceptable ester or salt thereof and
(b) 2′,3′-dideoxycytidine
or a pharmaceutically acceptable ester or salt thereof, components (a) and (b) of the combination being employed together such that a synergistic antiviral effect is achieved. The term "synergistic antiviral effect" is used to denote an antiviral effect which is greater than the predicted purely additive effects of the individual components (a) and (b) of the combination.

A preferred combination according to the invention is a combination of a compound of formula I with 2′,3′-dideoxycytidine.

Preferred esters according to the invention include carboxylic acid esters ; sulphonate esters ; mono-, di- or triphosphate esters ; and amino acid esters.

Examples of pharmaceutically acceptable salts include base salts e.g. derived from an appropriate base such as an alkali metal (e.g. sodium) or alkaline earth metal (e.g. magnesium) salts.

It should be noted that the invention includes the optical and geometric isomers and all tautomeric forms of the components (a) and (b) of the combination, either alone or in admixture.

Examples of viral infections and associated clinical conditions which may be treated or prevented in accordance with the invention, include human retroviral infections such as human immunodeficiency virus (HIV), e.g. HIV-1 or HIV-2, and human T-cell lymphotropic virus (HTLV) e.g. HTLV-I or HTLV-II infections. The combinations of the present invention are especially useful for the treatment or prophylaxis of AIDS and related clinical conditions such as AIDS-related complex (ARC), progressive generalized lymphadenopathy (PLG), AIDS-related neurological conditions, such as multiple sclerosis or tropical paraparesis, anti-HIV antibody-positive patients, and HIV-positive conditions such as Kaposi's sarcoma and thrombocytopenic purpura. The combinations of the present invention may also be used in the treatment of prevention of psoriasis.

The combination of the present invention may also be used in the treatment or prophylaxis of hepatitis B virus infections and associated clinical conditions.

The combinations of the present invention have been found to be particularly applicable to the treatment of asymptomatic infections or diseases in humans caused by or associated with human retroviruses.

According to a second feature of the invention there are provided combinations as hereinbefore described, for use in medical therapy, particularly for the treatment or prophylaxis of any of the aforementioned viral infections or conditions, especially HIV infections including AIDS.

The present invention further includes a process for preparing the combinations hereinbefore described, which comprises bringing into association components (a) and (b) of the combination in a medicament to provide a synergistic antiviral effect.

In a further aspect of the present invention, there is provided the use of a combination of the present invention in the manufacture of a medicament for the treatment or prophylaxis of any of the aforementioned viral infections or conditions.

It will be appreciated that in accordance with the present invention, components (a) and (b) of the combination may be administered simultaneously or sequentially. In the latter case, however, the components are administered within a sufficiently short interval to ensure that a synergistic antiviral effect is achieved.

An advantage of the combination of the present invention is that it enables attainment of an improved antiviral efficacy at a particular dose of one of the antiviral components (compared with the component used alone) thereby improving the therapeutic index of the component. Thus, for example, the combination may be used to treat conditions which would otherwise require relatively large doses of the antiviral component at which toxicity problems may occur. The smaller doses of the combination may provide increased convenience to the patient and increased compliance.

The combinations of the present invention may be administered to a mammal (the "recipient") in a conventional manner. As indicated above, components (a) and (b) may be administered simultaneously (e.g., in a unitary pharmaceutical formulation) or separately (e.g., in separate pharmaceutical formulations). In general, the combinations may be administered by the topical, oral, rectal or parenteral (e.g., intravenous, subcutaneous or intramuscular) route. It will be appreciated that the route may vary with, for example, the severity of the condition to be treated and the identity of the recipient.

The dose of the combination will depend on the condition being treated and other clinical factors such as the weight and condition of the recipient and the route of administration of the compound. Examples of dose ranges and component ratios are as follows.

The ratios of 2′,3′-dideoxynucleoside to carbovir, in μmoles, for use according to this invention are from 1: 50 preferably from 1 : 10 and most preferably 1 : 2.

Hereafter the components of the combination may be referred to as "active ingredients".

In general a suitable dose of a combination of the present invention will be in the range of 0.10 to 10 mg per kilogram body weight of the recipient per day, preferably in the range of 0.25 to 6.0 mg per kilogram body weight per day and most preferably in the range 0.20 to 4.0 mg per kilogram body weight per day. The desired dose is preferably presented as two, three, four, five, six or more sub-doses administered at appropriate intervals throughout the day. These sub-doses may be administered in unit dose forms, for example, containing 1 to 150 mg, preferably 2 to 100 mg, and most preferably 5 to 70 mg of active ingredients per unit dose form.

While it is possible for the active ingredients to be administered alone it is preferable to present them as a pharmaceutical formulation. The formulations of the present invention comprise a combination according to the invention together with one or more acceptable carriers and optionally other therapeutic agents. Each carrier must be "acceptable" in the sense of being compatible with the other ingredients of the formulation and not injurious to the patient. Formulations include those suitable for oral, rectal, nasal, topical (including buccal and sublingual), vaginal or parenteral (including subcutaneous, intramuscular, intravenous and intradermal) administration. The formulations may conveniently be presented in unit dosage form and may be prepared by any methods well known in the art of pharmacy. Such methods include the step of bringing into association the active ingredients with the carrier which constitutes one or more accessory ingredients.

In general, the formulations are prepared by uniformly and intimately bringing into association the active ingredients with liquid carriers or finely divided solid carriers or both, and then if necessary shaping the product.

Formulations of the present invention suitable for oral administration may be presented as discrete units such as capsules, cachets or tablets each containing a predetermined amount of the active ingredients ; as a powder or granules ; as a solution or a suspension in an aqueous or non-aqueous liquid ; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredients in a free-flowing form such as a powder or granules, optionally mixed with a binder (e.g. povidone, gelatin, hydroxypropylmethyl cellulose), lubricant, inert diluent, preservative, disintegrant (e.g. sodium starch glycollate, cross-linked povidone, cross-linked sodium carboxymethyl cellulose) surface-active or dispersing agent. Moulded tablets may be made by moulding a mixture of the powdered compound moistened with an inert liquid diluent in a suitable machine. The tablets may optionally be coated or scored and may be formulated so as to provide slow or controlled release of the active ingredients therein using, for example, hydroxypropylmethyl cellulose in varying proportions to provide the desired release profile. Tablets may optionally be provided with an enteric coating, to provide release in parts of the gut other than the stomach. This is particularly advantageous for purine nucleoside derivatives as such compounds are susceptible to acid hydrolysis.

Formulations suitable for topical administration in the mouth include lozenges comprising the active ingredients in a flavoured base, usually sucrose and acacia or tragacanth ; pastilles comprising the active ingredient in an inert basis such as gelatin and glycerin, or sucrose and acacia ; and mouthwashes comprising the active ingredient in a suitable liquid carrier. Formulations for rectal administration may be presented as a suppository with a suitable base comprising for example cocoa butter or a salicylate.

Topical administration may also be by means of a transdermal iontophoretic device.

Formulations suitable for vaginal administration may be presented as passaries, tampons, creams, gels, pastes, foams or spray formulations containing in addition to the active ingredient such carriers as are known in the art to be appropriate.

Formulations suitable for parenteral administration include aqueous and nonaqueous isotonic sterile injection solutions which may contain anti-oxidants, buffers, bacteriostats and solutes which render the formulation isotonic with the blood of the intended recipient ; and aqueous and non-aqueous sterile suspensions which may include suspending agents and thickening agents, and liposomes or other microparticulate systems which are designed to target the compound to blood components or one or more organs. The formulations may be presented in unit-dose or multi-dose sealed containers, for example, ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets of the kind previously described.

Preferred unit dosage formulations are those containing a daily dose or daily subdose of the active ingredients, as hereinbefore recited, or an appropriate fraction thereof.

The compounds according to the invention may also be presented for use in the form of veterinary compositions, which may be prepared, for example, by methods that are conventional in the art.

It should be understood that in addition to the ingredients particularly mentioned above the formulations of this invention may include other agents conventional in the art having regard to the type of formulation in question, for example, those suitable for oral administration may include such further agents as sweeteners, thickeners and flavoring agents.

The components of the combination of the present invention may be prepared in conventional manner for example as described hereinafter.

DDC can be prepared in conventional manner, for example, as described in Hortwitz et al., J. Org. Chem. 32 (3), 817-18 1967) and Lin et al., J. Med. Chem. 30 (2), 440-444 (1987). DDC can also be obtained from Aldrich Chemical Co., Milwaukee, WI 53233.

Carbovir can be synthesized by conversion of cis-4-acetamidocyclopent-2-enemethyl acetate (Daluge and Vince, J. Org. Chem. 43, 2311 (1978)) to (+ –)-9-(cis-4-hydroxymethyl)-2-cyclopentenyl) guanine by the method of Shealy and Clayton, J. Pharm. Sci., 62, 1432 (1973).

Salts and esters of the component compounds may be prepared in conventional manner.

The following examples are intended for illustration.

Example 1 : Tablet Formulations

The following formulations A, B and C are prepared by wet granulation of the ingredients with a solution of povidone, followed by addition of magnesium stearate and compression.

Formulation A

| | mg/tablet |
|---|---|
| Component (a) | 4 |
| Component (b) | 2 |
| Lactose B.P. | 210 |
| Povidone B.P. | 15 |
| Sodium Starch Glycollate | 20 |
| Magnesium Stearate | 5 |
| | 256 |

Formulation B

| | mg/tablet |
|---|---|
| Component (a) | 4 |
| Component (b) | 2 |
| Lactose B.P. | 150 |
| Avicel® PH 101 | 60 |
| Povidone B.P. | 15 |
| Sodium Starch Glycollate | 20 |
| Magnesium Stearate | 5 |
| | 256 |

Formulation C

| | mg/tablet |
|---|---|
| Component (a) | 2 |
| Component (b) | 1 |
| Lactose B.P. | 200 |
| Starch | 50 |
| Povidone | 5 |
| Magnesium Stearate | 4 |
| | 262 |

The following formulations, D and E, are prepared by direct compression of the admixed ingredients. The lactose in formulation E is of the direct compression type (Dairy Crest — "Zeparox").

Formulation D

| | mg/tablet |
|---|---|
| Component (a) | 4 |
| Component (b) | 2 |
| Pregelatinized Starch NF15 | 150 |
| | 156 |

Formulation E

| | mg/tablet |
|---|---|
| Component (a) | 4 |
| Component (b) | 2 |
| Lactose B.P. | 150 |
| Avicel® | 100 |
| | 256 |

Formulation F (Controlled Release Formulation)

The formulation is prepared by wet granulation of the ingredients with a solution of povidone followed by the addition of magnesium stearate and compression.

| | mg/tablet |
|---|---|
| Component (a) | 4 |
| Component (b) | 2 |
| Hydroxypropylmethylcellulose (Methocel® K4M Premium) | 112 |
| Lactose B.P. | 53 |
| Povidone B.P. | 28 |
| Magnesium Stearate | 7 |
| | 206 |

Drug release takes place over a period of about 6-8 hours and is complete after 12 hours.

Example 2 : Capsule Formulations

Formulation A

A capsule formulation is prepared by admixing the ingredients of Formulation D in Example 1 above and filling into a two-part hard gelatin capsule. Formulation B (infra) is prepared in a similar manner.

Formulation B

| | mg/capsule |
|---|---|
| Component (a) | 4 |
| Component (b) | 2 |
| Lactose B.P. | 143 |
| Sodium Starch Glycollate | 25 |
| Magnesium Stearate | 2 |
| | 176 |

Formulation C

| | mg/capsule |
|---|---|
| Component (a) | 4 |
| Component (b) | 2 |
| Macrogol® 4000 B.P. | 350 |
| | 356 |

Capsules of formulation C are prepared by melting the Macrogol® 4000 B.P., dispersing the active ingredients in the melt and filling the melt into a two-part hard gelatin capsule.

Formulation D

| | mg/capsule |
|---|---|
| Component (a) | 4 |
| Component (b) | 2 |
| Lecithin | 100 |
| Arachis Oil | 100 |
| | 206 |

Capsules of formulation D are prepared by dispersing the active ingredients in the lecithin and arachis oil and filling the dispersion into soft, elastic gelatin capsules.

Formulation E (Controlled Release Capsule)

The following controlled release capsule formulation is prepared by extruding ingredients a, b, c and d using an extruder, followed by spheronization of the extrudate and drying. The dried pellets are then coated with release-controlling membrane (e) and filled into a two-piece, hard gelatin capsule.

| | mg/capsule |
|---|---|
| (a) Component (a) | 4 |
| (b) Component (b) | 2 |
| (c) Microcrystalline Cellulose | 125 |
| (d) Lactose B.P. | 125 |
| (e) Ethyl Cellulose | 13 |
| | 269 |

Example 3 : Injectable Formulation

Formulation A

| | |
|---|---|
| Component (a) | 4 mg |
| Component (b) | 2 mg |
| Hydrochloric acid solution 0.1M or | |
| Sodium hydroxide solution 0.1M q.s. to pH | 4.0 to 7.0 |
| Sterile water q.s. to | 10 ml |

The active ingredients are dissolved in most of the water (35°-40°C) and the pH adjusted to between 4.0 and 7.0 with the hydrochloric acid or the sodium hydroxide as appropriate. The batch is then made up to volume with the water and filtered through a sterile micropore filter into a sterile 10 ml amber glass vial (type 1) and sealed with sterile closures and overseals.

Formulation B

| | |
|---|---|
| Ccmponent (a) | 2 mg |
| Component (b) | 1 mg |
| Sterile, pyrogen-free, pH 7 phosphate buffer, q.s. to | 25 ml |

Example 4 : Intramuscular injection

| | |
|---|---|
| Component (a) | 4 mg |
| Component (b) | 2 mg |
| Benzyl Alcohol | 0.10 g |
| Glycofurol 75 | 1.45 g |
| Water for Injection q.s. to | 3.00 ml |

The active ingredients are dissolved in the glycofurol. The benzyl alcohol is then added and dissolved, and water added to 3 ml. The mixture is then filtered through a sterile micropore filter and sealed in sterile 3 ml amber glass vials (type 1).

Example 5 : Syrup

| | |
|---|---|
| Component (a) | 4 mg |
| Component (b) | 2 mg |
| Sorbitol Solution | 1.50 g |
| Glycerol | 2.00 g |
| Sodium Benzoate | 0.005 g |
| Flavor, Peach 17.42.3169 | 0.0125 ml |
| Purified Water q.s. to | 5.00 ml |

The active ingredients are dissolved in a mixture of the glycerol and most of the purified water. An aqueous solution of the sodium benzoate is then added to the solution, followed by addition of the sorbitol solution and

finally the flavor. The volume is made up with purified water and mixed well.

Example 6 : Suppository

| | mg/suppository |
|---|---|
| Component (a) (63 μm)* | 4 |
| Component (b) (63 μm)* | 2 |
| Hard Fat, BP (Witepsol® H15 - Dynamit Nobel) | 1770 |
| | 1776 |

*The active ingredients are used as a powder wherein at least 90% of the particles are of 63 μm diameter or less.

One-fifth of the Witepsol® H15 is melted in a steam-jacketed pan at 45°C maximum. The active ingredients are sifted through a 200 μm sieve and added to the molten base with mixing, using a silverson fitted with a cutting head, until a smooth dispersion is achieved. Maintaining the mixture at 45°C, the remaining Witepsol® H15 is added to the suspension and stirred to ensure a homogenous mix. The entire suspension is passed through a 250 μm stainless steel screen and, with continuous stirring, is allowed to cool to 40°C. At a temperature of 38°C to 40°C, 2.02 g of the mixture is filled into suitable, 2 ml plastic moulds. The suppositories are allowed to cool to room temperature.

Example 7 : Pessaries

| | mg/pessary |
|---|---|
| Component (a) (63 μm) | 4 |
| Component (b) (63 μm) | 2 |
| Anhydrate Dextrose | 380 |
| Potato Starch | 363 |
| Magnesium Stearate | 7 |
| | 756 |

The above ingredients are mixed directly and pessaries prepared by direct compression of the resulting mixture.

Example 8 : Preparation of (+ –)-9-(cis-4-(hydroxymethyl)-2-cyclopentenyl)quanine(carbovir)

A. (+ –)4-(1α,4α)-[(2-Amino-4-chloro-6-pyrimidinyl)amino]-2-cyclopentene-1-methanol

cis-4-Acetamidocyclopent-2-enemethyl acetate [Daluge and Vince, J. Org. Chem., 43, 2311 (1978)] (14.88 g, 0.073 mole) and barium hydroxide octahydrate (46.19 g, 0.146 mole) were refluxed in water (300 ml) under nitrogen for 18 hours. The resulting solution was neutralized with carbon dioxide. The precipitate was washed with water, then ethanol.

The combined filtrate-wash was evaporated to a syrup (11.16 g) which was condensed with 2-amino-4,6-dichloropyrimidine (23.91 g, 0.146 mole) and triethylamine (30.5 ml, 0.219 mole) in refluxing 1-butanol (100 ml) for 1.5 hours. After addition of 1 N Sodium hydroxide (73 ml), the resulting mixture was evaporated to dryness and the residual solid slurried in chloroform (200 ml). Unreacted 2-amino-4,6-dichloropyrimidine was filtered off and washed with chloroform (100 ml). The chloroform filtrate-wash was concentrated and chromatographed on a silica gel column. Additional pyrimidine starting material was eluted with 2.5% methanol-chloroform. The title compound was eluted with — 3.5% methanol-chloroform as an off-white solid foam

(15.90 g, 91%) ; 'H-NMR ($Me_2SO$-d6) δ 1.15-1.28 and 2.26-2.41 (2 m, 2, $CH_2$), 2.60-2.71 (m, 1, 1'-H), 3.4 (m, overlapping $H_2O$, $CH_2OH$), 4.625 (t, J = 5.3, 1, $CH_2OH$), 4.95 (br s, 1, CH-N), 5.67-5.87 (m, 2, CH=CH and pyrimidine H-5), 6.38 (br s 1, NH2), 7.12 (br s, 1, NH) ; MS (CI) M + 1 : 241, 243.

Anal. Calcd. for $C_{10}H_{13}N_4OCl \cdot O \cdot 2H_2O$ : C, 48.99 ; H, 5.55 ; N, 22.85 ; Cl, 14.46. Found : C, 49.10 ; H, 5.57; N, 22.81 ; Cl, 14.40.

B.(+ –)-(1α,4α)-4-[[2-Amino-6-chloro-5-[4-chlorophenyl)azo]-4-pyrimidinyl]amino]-2-cyclopentene-1-methanol

(+ –)4-(1α,4α)-[(2-Amino-4-chloro-6-pyrimidinyl)amino]-2-cyclopentene-1-methanol(11.58 g, 48.1 mmole) and sodium acetate trihydrate (97 g) were dissolved in glacial acetic acid (225 ml) and water 225 ml). A cold solution (0-5°C) of 4-chlorobenzenediazonium chloride was prepared from 4-chloroaniline (6.74 g, 52.8 mmole), concentrated hydrochloric acid (14.7 ml), water (52 ml), and sodium nitrite (4.01 g, 58.2 mmol in 47 ml of water). This cold solution was added dropwise over 5 minutes to the first solution. The resulting yellow precipitate was filtered after 18 hours, washed with water, and extracted with ethanol to give the title compound as a dark yellow powder (12.56 g, 69%), mp 218-220°C dec : 'H-NMR ($Me_2SO$-d6) δ 10.25 (d, 1, NH), 7.69 and 7.54 (both d, J = 8.9,) overlapping 7.6 (br, 6, $C_6H_4$ and $NH_2$), 5.80-5.95 (m, 2, CH=CH), 5.24 (m, 1, CHN), 4.75 (t, 1, $CH_2OH$), 3.41 (t, 2, $CH_2OH$), 2.75 (m, 1, CH), 2.41 (m, 1, CH), 1.44-1.53 (m, 1, CH).

Anal Calcd. for $C_{16}H_{16}N_6Cl_2O$ : C, 50.67 ; H, 4.25 ; N, 22.16 ; Cl, 18.70. Found : C, 50.59 ; H, 4.29 ; N, 22.10 ; Cl, 18.66

C. (+ –)-(1α,4α)-[(2,5-Diamino-4-chloro-6-pyrimidinyl)-amino]-2-cyclopentene-1-methanol

The title compound of Example 8B (11.67 g) was suspended in ethanol (235 ml), glacial acetic acid (30 ml), and water 235 ml). The mixture was heated to reflux under nitrogen. Zinc dust (13.5 g) was added in small portions over 30 minutes during which time the compound dissolved. The reaction was heated an additional 20 minutes, and then the excess zinc was filtered off from the hot solution, and was washed with ethanol. The filtrates were evaporated, and the residue was purified on a silica gel column eluting with chloroform (1 L) and chloroform : methanol/4 : 1 (1.8 L). The fractions containing the product were combined, and the solvent was removed under reduced pressure to give the title compound as a red-orange oil (11.2 g, > 100% yield). A pure sample was obtained during another small scale reaction to obtain the product as a light yellow solid in a 76% yield ; 1H-NHR ($Me_2SO-d_6$) δ 1.29 and 2.39 (m, 2, $CH_2$), 2.69 (t, 1, 1'-H), 3.37 (d, 2, $\underline{CH_2}OH$), 3.91 (br, 2 $NH_2$), 4.60 (br, 1, $CH_2\underline{OH}$), 5.02 (m, 1, $\underline{C}HNH$), 5.56 (br s, 2, $NH_2$), 5.74 (m, 1, =CH), 5.86 (m, 1, =CH), 6.36 (d, 1, $CH\underline{NH}$).

D. (+ –)-(1α,4α)-4-(2-Amino-6-chloro-9H-purin-9-yl)-2-cyclopentene-1-methanol

The title compound of Example 8C (~ 9.7 g) was dissolved in diethoxymethyl acetate (100 g), and refluxed for two days. The solvent was removed under high vacuum at 50°C, and dioxane (40 ml) and 0.5 N hydrochloric acid (60 ml) was added. The reaction was stirred at room temperature for 1.25 hours, and then chilled. The reaction was neutralized to pH 7 with cold 5 N sodium hydroxide, and then it was extracted with choroform : methanol/3 : 1 several times. The organic layers were dried with magnesium sulfate, filtered, and evaporated. The residus was purified by chromatography on a silica gel column, eluting with 2% methanol/chloroform to give 3.7 g (46% yield) of the title compound, mp 138-139°C ; 1H-NMR ($Me_2SO-d_6$) δ 1.63 and 2.61 (m, 2, $CH_2$), 2.87 (m, 1, 1'-H), 3.44 (m, 2, $CH_2OH$), 5.44 (m, 1, CH-N), 5.89 (m, 1, =CH), 6.14 (m, 1, =CH), 6.82 (br s, 2, $NH_2$), 8.02 (s, 1, 8-H). UV : pH 1 λmax 315 (ε 7370), 218 (26200) ; λsh 239.5 (5650) ; pH 7.4 λmax 307 (ε 8000), 245.5 (4600), 223 (26400). MS : (EI) 265, 267 (M+) : (CI) 266, 268 (M + 1).

Anal. Calcd. for $C_{11}H_{12}N_5C_{10} \cdot 2H_2O$ : C, 43.79 ; H, 5.35 ; N, 23.21 ; Cl, 11.75. Found : C, 43.67 ; H, 5.29 ; N, 23.05 ; Cl, 11.70.

E. (+ –)-9-[cis-4-(hydroxymethyl)-2-cyclopentenyl]quanine

(+ –)-(1α,4α)-4-(2-Amino-6-chloro-9H-purin-9-yl)-2-cyclopentene-1-methanol hydrochloride (0.57 g, 1.89 mmol) and 1 N hydrochloric acid (45 ml) were heated at 80-90°C for 15 min. The resulting dark solution was neutralized with 1 N sodium hydroxide, treated with charcoal and filtered while hot. The filtrate deposited the title compound as white crystals (167 mg, 29%). A second crystallization from water gave a white powder ; mp > 320°C.

Anal. Calcd. for $C_{11}H_{13}N_5O_2 \cdot H_2O$ : C, 49.81 ; H, 5.70 ; N, 26.40. Found : C, 49.89 ; H, 5.71 ; N, 26.33. 1H-NMR ($Me_2SO-d_6$) δ 10.54 (br s, 1, NHC=O), 7.59 (s, 1, H-8), 6.43 (br s, 2, $NH_2$), 6.1 and 5.8 (both m, 2, CH=CH),

5.3 (m, 1, CH-N), 4.73 (t, J = 5.3, 1, OH), 3.43 (t, J = 5.6, 2, $CH_2$-O), 2.8-2.6 (m, 1, H-4′), 2.65-2.55 and 1.6-1.5 (both m, 2, $CH_2$).

Example 9 : Preparation of 2′,3′-dideoxycytidine (DDC)

Acetic anhydride (0.96 g, 9.45 mmol) was added slowly to a stirred solution of 2′,3′-dideoxyuridine (Aldrich Chemical Co., Milwaukee, WI 53233) (0.40 g, 1.89 mmol) in 10 mL of pyridine at 0°C (ice bath). The resultant solution was allowed to stand overnight at 4°C. The solvent and the excess acetic anhydride was removed in vacuo. The remaining residue was dissolved in 50 mL of chloroform and washed in a separatory funnel with 50 mL portions of water (three times), saturated sodium hydrogen carbonate (two times), and water again (two times). The chloroform solution was clarified with Norit, dried with anhydrous magnesium sulphate, and filtered. The filtrate was then concentrated to a residue, which was used immediately without further purification for the next preparation.

The acetate was dissolved in 10 mL of pyridine. While the mixture was stirred in a cold-water bath, 4-chlorophenyl phosphorodichloridate (0.70 g 2.84 mmol) was added dropwise, followed by the addition of 1,2,4-triazole (0.39 g, 5.68 mmol). The mixture was stirred at room temperature for 3 days and then concentrated under reduced pressure. The resulting residue was dissolved in 25 mL of dichloromethane and washed with 25 mL of water (two times) and 50% sodium hydrogen carbonate solution (25 mL). The dichloromethane solution was clarified with Norit, dried (magnesium sulphate), and filtered. The filtrate was evaporated to dryness in vacuo to a yield a glassy residue (4-triazolylpyrimidinone derivative), which was dissolved in 20 mL of ammonium hydroxide/dioxane (1 : 3). The mixture was stirred for 5 hours at room temperature in a Wheaton pressure bottle. This solution was then concentrated and the remaining residue was stirred overnight in the pressure bottle at room temperature in 20 mL of saturated methanolic ammonia. The solution was then reduced to a small volume in vacuo and chromatographed on a silica gel column (chloroform/methanol, 3 : 1, Rf 0.4) to afford 0.18 g of product : mp 209-210°C.

Antiviral Activity

Combinations according to the invention were tested for anti-HIV activity in an HIV-infected MT4 cell assay. The cells were exposed to HIV for one hour prior to addition of antiviral component(s). Components were tested in serial 2.5-fold dilutions. After five days of incubation at 37°C, the cell number was determined. Inhibition of HIV-induced cytopathic effect was calculated, and synergism was determined by FIC plots as described by Elion, Singer, and Hitchings, J. Biol. Chem., 208, 477 (1954).

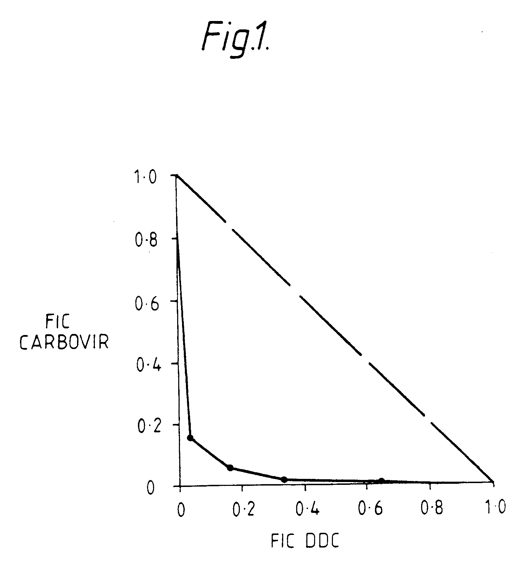

The fractional inhibitor concentration (FIC) of carbovir is plotted against that of the 2′,3′-dideoxynucleoside (Table 1). When points fall beneath the dotted line, synergy between the two components is indicated (Figure 1). When data points fall on the dotted line, the activities of the two components are additive. Points above the line indicate antagonism.

As can be seen in Figure 1, the combination of carbovir and DDC is strongly synergistic.

Table 1

Calculation of Fractional Inhibitor

Concentration (FIC)

80% Inhibition

| DDC (μM) | Carbovir (μM) | $FIC_{DDC}$ | $FIC_{carbovir}$ |
|---|---|---|---|
| 0.3 | 8 | 0.3/9 = 0.03 | 8/50 = 0.16 |
| 1.5 | 3.2 | 1.5/9 = 0.16 | 2.2/50 = 0.06 |
| 3 | 1.3 | 3/9 = 0.33 | 1.3/50 = 0.02 |
| 5.8 | 0.5 | 5.8/9 = 0.64 | 0.5/50 = 0.01 |
| 9 | - | | |
| - | 50 | | |

**Claims**

1. A combination of :
(a) (+ –)-9-(cis-4-(hydroxymethyl)-2-cyclopentenyl)guanine or a pharmaceutically acceptable ester or salt thereof, and
(b) 2′,3′-dideoxycytidine
or a pharmaceutically acceptable ester or salt thereof, component (a) and (b) of the combination being employed together such that a synergistic antiviral effect is achieved.

2. A combination as claimed in claim 1 wherein component (a) is (+ –)-9-(cis-4-(hydroxymethyl)-2-cyclopentenyl)guanine.

3. A combination as claimed in claim 1, wherein component (b) is 2′,3′-dideoxycytidine.

4. A combination as claimed in claims 1, 2 or 3 wherein component (a) is (+ –)-9-(cis-4-(hydroxymethyl)-2-cyclopentenyl)guanine and component (b) is 2′,3′-dideoxycytidine.

5. A pharmaceutical formulation comprising a combination as claimed in any of claims 1 to 4 together with at least one pharmaceutical carrier.

6. A pharmaceutical formulation as claimed in claim 5 in the form of a tablet or capsule.

7. A combination as claimed in any of claims 1 to 4 for use in medical therapy.

8. A combination as claimed in claim 7 for use in the treatment or prophylaxis of human immunodeficiency virus infections.

9. A combination as claimed in claim 8 for use in the treatment or prophylaxis of acquired immune deficiency

syndrome.

10. Use of a combination as claimed in any of claims 1 to 5 in the manufacture of a medicament for the treatment or prophylaxis of human immunodeficiency virus infections.

11. A combination as claimed in claim 7 for use in the treatment or prophylaxis of hepatitis B virus infections.

## Patentansprüche

1. Kombination von :

(a) (+ –)-9-(cis-4-(Hydroxymethyl)-2-cyclopentenyl)-guanin oder ein pharmazeutisch annehmbarer Ester oder Salz hievon, und

(b) 2′,3′-Didesoxycytidin oder ein pharmazeutisch annehmbarer Ester oder Salz hievon, wobei die Komponenten (a) und (b) der Kombination zusammen verwendet werden, wodurch eine synergistische antivirale Wirkung erzielt wird.

2. Kombination nach Anspruch 1, wobei die Komponente (a) (+ –)-9-(cis-4-(Hydroxymethyl)-2-cyclopentenyl)-guanin ist.

3. Kombination nach Anspruch 1, wobei die Komponente (b) 2′,3′-Didesoxycytidin ist.

4. Kombination nach Anspruch 1, 2 oder 3, wobei die Komponente (a) (+ –)-9-(cis-4-(Hydroxymethyl)-2-cyclopentenyl)-guanin und die Komponente (b) 2′,3′-Didesoxycytidin ist.

5. Pharmazeutische Formulierung, umfassend eine Kombination nach einem der Ansprüche 1 bis 4 zusammen mit wenigstens einem pharmazeutischen Träger.

6. Pharmazeutische Formulierung nach Anspruch 5 in Form einer Tablette oder Kapsel.

7. Kombination nach einem der Ansprüche 1 bis 4 zur Verwendung in der medizinischen Therapie.

8. Kombination nach Anspruch 7 zur Verwendung bei der Behandlung oder Prophylaxe von Immunschwächevirusinfektionen beim Menschen.

9. Kombination nach Anspruch 8 zur Verwendung bei der Behandlung oder Prophylaxe von erworbenem Immunschwächesyndrom.

10. Verwendung einer Kombination nach einem der Ansprüche 1 bis 5 bei der Herstellung eines Medikaments zur Behandlung oder Prophylaxe von Immunschwächevirusinfektionen beim Menschen.

11. Kombination nach Anspruch 7 zur Verwendung bei der Behandlung oder Prophylaxe von Hepatitis-B-Virusinfektionen.

## Revendications

1. Mélange :

(a) de (+ –)-9-(cis-4-(hydroxyméthyl)-2-cyclopentényl)guanine ou d'un sel ou ester pharmaceutiquement acceptable de celle-ci, et

(b) de 2′,3′-didésoxycytidine ou d'un sel ou ester pharmaceutiquement acceptable de celle-ci,

les composants (a) et (b) du mélange étant utilisés ensemble de façon qu'un effet antiviral synergique soit atteint.

2. Mélange suivant la revendication 1, dans lequel le composant (a) est la (+ –)-9-(cis-4-(hydroxyméthyl)-2-cyclopentényl)guanine.

3. Mélange suivant la revendication 1, dans lequel le composant (b) est la 2′,3′-didésoxycytidine.

4. Mélange suivant la revendication 1, 2 ou 3, dans lequel le composant (a) est la (+ –)-9-(cis-4-(hydroxyméthyl)-2-cyclopentényl)guanine et le composant (b) est la 2′,3′-didésoxycytidine.

5. Composition pharmaceutique comprenant un mélange suivant l'une quelconque des revendications 1 à 4 conjointement avec au moins un excipient pharmaceutique.

6. Composition pharmaceutique suivant la revendication 5, sous la forme d'un comprimé ou d'une capsule.

7. Mélange suivant l'une quelconque des revendications 1 à 4, à utiliser en thérapeutique médicale.

8. Mélange suivant la revendication 7, à utiliser pour le traitement ou la prophylaxie d'infections par le virus de l'immunodéficience humaine.

9. Mélange suivant la revendication 8, à utiliser pour le traitement ou la prophylaxie du syndrome de l'immunodéficience acquise.

10. Utilisation d'un mélange suivant l'une quelconque des revendications 1 à 5 dans la préparation d'un médicament pour le traitement ou la prophylaxie d'infections par le virus de l'immunodéficience humaine.

11. Mélange suivant la revendication 7, à utiliser dans le traitement ou la prophylaxie d'infections par le virus de l'hépatite B.

*Fig.1.*